# EUROPEAN PATENT APPLICATION

(11) **EP 2 202 521 A1**
(43) Date of publication of application: **30.06.2010**
(21) Application number: 10154491.4
(22) Date of filing: 15.01.2002
(51) Int. Cl.: G01N 33/543, G01N 21/55

(54) **Surface chemical modification of optical elements for the spectroscopic detection of molecules and organic components**

(30) Priority: 22.03.2001 US 278028 P; 16.01.2001 EP 01870008
(62) Divisional of application: 02716660.2
(71) Applicant: Université Catholique De Louvain, 1348 Louvain-La-Neuve (BE); Université Libre de Bruxelles, 1070 Bruxelles (BE); Université de Mons-Hainaut, 7000 Mons (BE)
(72) Inventor: Marchand-Brynaert, Jacqueline Anne-Marie Germaine, 1348 Louvain-La Neuve (BE); Goormaghtigh, Erik Robert Marcel Charles, 1070 Bruxelles (BE); Homblé, Fabrice Roland, 1070 Bruxelles (BE); Voué, Michel Pierre Ernest, 7000 Mons (BE); De Coninck, Joël Joseph Florent, 7000 Mons (BE)
(74) Representative: Bounaga, Sakina

(57) **Abstract**

The invention relates to a device suitable for the investigation of ligand-receptor interactions, in particular for the investigation of an analyte target interaction such as biological and chemical molecules and organic components and their interaction with surfaces, consisting of an attenuated total internal reflection element, transparent in the infra-red and of which at least one surface is chemically activated and covalently grafted with an organic molecule able to immobilize the receptor.

The invention further relates to the use of said device and a method for the construction of said device comprising the steps of:
- surface activation of at least one surface of an attenuated total internal reflection element,
- surface grafting with an organic molecule of the activated surface obtained in the previous step, and
- coupling a receptor via covalent fixation on the organic molecule.

## Description

The invention relates to devices suitable for the investigation of ligand-receptor interactions, in particular for the investigation of biological or chemical molecules and organic components and their interaction with suitably modified surfaces.

More in particular the invention concerns methods of chemical surface activation and covalent grafting of ATR (Attenuated Total Internal Reflection) elements and their use in FTIR (Fourier Transform Infra Red) devices for the detection, characterization, and dosage of biological molecules and organic compounds.

In another aspect the invention also relates to the grafted ATR elements as such.

### Background of Art

Biosensors (for a detailed review, see: Leech D, Chem. Soc. Rev., 1994, 23, 205-213) are devices based on the specific recognition of an analyte of interest by a target such as a biological component, for example a receptor, an antibody, an enzyme, a membrane, a cell or cell containing media , a molecule and the subsequent transformation of this interaction into an electrical, optical, or other signal. Biosensors have already attracted intensive interest in many different fields such as medical diagnostics and control, environmental analysis, and monitoring of biotechnological processes.

Different surface sensitive techniques can be applied to detect ligand-receptor interactions, depending on the nature of the sensor supports. They are piezoelectric methods, impedance spectroscopy, microscopy, and surface plasmon resonance (SPR) spectroscopy, in the case of gold or other metal surfaces, on the one hand, and integrated optics and fluorescence spectroscopy, in the case of glass (or TiO₂) surfaces, on the other hand.

Amongst the previous techniques, SPR spectroscopy has been successfully used by BIACORE International AB (Stockholm) to develop a commercial instrument (Löfas S, Malmqvist M, Rönnberg I, Stenberg E, Liedberg B, Lundström I, Sensors Actuators B, 1991, 5, 79; Malmqvist M, Nature, 1993, 361, 186). Surface plasmon resonance is a surface sensitive technique able to probe molecular interactions in real time, and on-line (for selected examples, see: Kooyman RPH, de Bruijn HE, Eenink RG, Greve J, J. Mol. Struct., 1990, 218, 345; Liedburg B, Nylander C, Lundström I, Sensors Actuators B, 1983, 4, 299; Karlsson R, Michaelsson A, Mattsson L, J. Immunol. Methods, 1991, 145, 229; Green RJ, Davies J, Davies MC, Roberts CJ, Tendler SJB, Biomaterials, 1997, 18, 405; Liedberg B, Lundström I, Stenberg E, Sensors Actuators B, 1993, 11, 63; Lahiri J, Isaacs L, Tien J, Whitesides GM, Anal. Chem., 1999 , 71, 777).

Biosensors based on the SPR optical technique make use of the changes in the refractive index of a medium near a thin film of metal (gold) deposited on a substrate (glass). Modifications in refractive index occur when an analyte such as a molecule or a protein, for instance is adsorbed or fixed to the surface substrate; consequently, the angle of minimum intensity of reflected light (the resonance angle) is affected. Intrinsically, this detection technique, i.e. measuring mass loading of the surface cannot provide structural and mechanistic information about the interacting analyte. The access to chemical information, such as molecular structure, packing, orientation,... and time-resolved information, such as conformational transitions accompanying the ligand-receptor interactions, are practically inaccessible. Moreover, the SPR technique requires the deposition of a metal film, which is usually gold on a support, and the subsequent immobilization of the targets or biological receptors of interest *via* the method of self-assembled monolayers (SAMs) based on the chemisorption of thiol-containing surfactants (for selected examples, see: Mrksich M, Whitesides GM, ACS Symp. Ser., 1997, 680, 361; Deng L, Mrksich M, Whitesides GM, J. Am. Chem. Soc., 1996, 118, 5136; Prime KL, Whitesides GM, J. Am. Chem. Soc., 1993, 115, 10714) or via the interaction with an amorphous Dextran matrix. The presence of this metal film (about 200 nm in depth) dramatically restricts the possibilities of surface detection by highly sensitive techniques, such as infra-red spectroscopy or fluorescence techniques that can be applied in aqueous media.-Accordingly, novel concepts and devices have to be found to improve the biosensor supports and the related detection techniques.

Fourier transform infra-red (FTIR) spectroscopy is an extremely powerful analytical technique, particularly well adapted to the characterization of organic molecules and biological systems. Quantitative structural and conformational information can be recorded. The method has been applied to the investigation of mono- and multilayers of bio-organic samples displayed on the optical elements, by using the attenuated total internal reflection (ATR) configuration (Scheuing DR), Fourier Transform Infra-Red Spectroscopy in Colloid and Interface Science, ACS Symposium Series n° 447, Am. Chem. Soc., Washington, 1991; Mirabella Jr FM (editor), Internal Reflection Spectroscopy, Theory and Applications, Marcel Dekker, New York, 1993). Interestingly, the ATR configuration allows the study of analytes such as biological components and molecules or proteins, for instance on surfaces in contact with water-containing media. The examination of protein adsorption on biomaterial surfaces is one of the relevant applications (Chittur KK, Biomaterials, 1998, 19, 357). Recently, Vogel and coworkers reported the effect of the reduction of the thickness of the metal layer deposited at the surface of an ATR element on the infrared detection of biomolecules (Liley, M.; Keller, T.A.; Duschl, C.; Vogel, H. Langmuir 1997, 13, 4190-4192). Later, an international patent application has appeared which discloses methods and devices for the detection and investigation of biological molecules at (or in) self-assembled monolayers on metal surfaces using infra-red spectroscopy in the attenuated total internal reflection configuration (Vogel H, Keller T, Liley M; WO 99/05509). This disclosure is based on the use of a very thin film of gold (5 to 30 nm) deposited on an ATR element, and the subsequent formation of SAMs susceptible to immobilize biomolecules. Thus, the relative transparency of the thin metal film in the infra-red is exploited, whereby the internal reflection of the IR beam at the interface between the ATR element and the metal produces an evanescent field which penetrates through the metal film and into the aqueous phase on the other side. This allows sampling of SAMs and fixed biomolecules at the metal-water interface.

Other techniques are available to immobilize molecules of interest on ATR crystals, such as the coating with thin layers of synthetic polymers, such as polyurethanes, polystyrene, polyethylene by spin-casting (Lenk TJ, Ratner BD, Chittur KK, Gendreau RM, Langmuir, 1991, 7, 1755; Lenk TJ, Ratner BD, Chittur KK, Gendreau RM, J. Biomed. Mater. Res., 1989, 23, 549), and the coating with bioceramic, such as hydroxyapatite by ion bombardment (Ong JL, Chittur KK, Lucas LC, J. Biomed. Mater. Res., 1994, 28, 1337). In all cases, i.e. metal, polymer or ceramic materials, the thickness of the deposited layer appears to be a crucial point because it is not allowed to significantly change the nature and the intensity of the evanescent field.

The main disadvantage of the use of an intermediate film of metal, polymer, or ceramic on the surface of the ATR element is the formation of a barrier for light transmission, which results in a drastic restriction of the possibilities to detect ligand-receptor interactions taking place at the ATR surface. Therefore, there is a further need for a method of surface modification of ATR elements which minimizes the effect of the deposition of an intermediate film of metal, polymer, or ceramic, between the crystal and the organic film displaying the biological receptors.

### Summary of the invention

In the present inventive application, the disturbing intermediate film is omitted and replaced by a modification of the ATR element surface involving a process consisting of two steps. The first step consists of chemically modifying the ATR element surface by wet chemistry. Preferably, ATR elements according to the invention are made of silicon or germanium crystals. The direct chemical modification of silicon surfaces by silanization methods is well known (Patai S and Rappoport Z (editors), The chemistry of Organic Silicon Compounds, John Wiley, Chichester, 1989; Mittal KL (editor), Silanes and other Coupling Agents, VSP, Utrecht, 1992; Auner N and Weis J (editors), Organosilicon Chemistry II, from Molecules to Materials, VCH, Weinheim, 1996); but this technique was never used for the elaboration of FTIR-ATR elements of biosensors.

The second step consists of grafting of the activated surface with an organic molecule, such as a silane derivative, through covalent coupling. The anchoring of a silane derivative through its silane moiety on the surface of an internal reflection element has been described previously (Stefan I and Scherson D, Langmuir, 2000, 16, 5945-5948). EP 0 596 421 discloses a coating of dielectric TiO₂ waveguides with elements capable of recognizing biological molecules in the formation of a biosensor. The coating consists of an organic support layer, to which the receptor molecules are bonded, the support layer comprising an ordered monomolecular layer which is bonded via a Si atom directly to a TiO₂ waveguide or if desired via an intermediate layer to a TiO₂ waveguide. However, none of these constructions disclose a chemical prior modification of the surface of the optical element. In present invention, pretreatment of the surface device by chemicals improves the wettability and the reactivity of the surface. Chemical activation of the ATR surface allows an easier and direct grafting of organic components on this surface. This results in a more accurate and efficient measuring of ligand-receptor interactions via infra-red spectroscopy.

The present invention is directed to a device suitable for the investigation of ligand-receptor interactions, in particular for the investigation of an analyte target interaction such as biological and chemical molecules and organic components and their interaction with surfaces, consisting of an attenuated total internal reflection element, transparent in the infra-red and of which at least one surface is chemically activated and covalently grafted with an organic molecule able to immobilize the receptor.

In a preferred embodiment the organic molecule is a silane derivative of the general formula

X₃Si - (CH₂)ₙ- (CF₂)_{n'}-Y, X₂(R₁)Si-(CH₂)ₙ-(CF₂)_{n'}-Y or

X(R₁)(R₂)Si-(CH₂)ₙ-(CF₂)_{n'}-Y,

wherein X is halogen, preferably Cl, Br or C₁-C₆ alkoxy, preferably OMe, OEt;
n is 1 to 20;
n' is 0 to 20;
R₁, R₂ are independently C₁-C₆ alkyl;
Y is Me, CF₃, CHF₂, CH₂F, CH=CH₂, CN, CH=O, epoxide, halogen, SH, NH₂,
OH, N=C=O, N=C=S, CO₂H or derived esters thereof.

In another preferred embodiment the organic molecule is a silane derivative covalently coupled with an multifunctional spacer-arm of the general formula

Z₁-(CH₂)ₙ-Z₂ wherein n is 2 to 12;

Z₁-CH₂-(O-CH₂-CH₂-)_{n'}-O-CH₂-Z₂ wherein n' is 0 to 5;

wherein Z₁,Z₂ are independently chosen from Aryl-N₃ (photoactivable substituents), CO₂H and activated forms thereof such as N-hydroxysuccinimidyl ester, CH₂NH₂ and activated derivatives such as N-maleimide, CH₂OH and activated forms such as tosylates, CH₂SH and activated forms such as dithiane derivatives, CH₂N=C=O or CH₂N=C=S.

The invention also provides for a method of construction of said device including the steps of:
- surface activation of at least one surface of an attenuated total internal reflection element,
- surface grafting with an organic molecule of the activated surface obtained in the previous step, and
- coupling a receptor via covalent fixation on the organic molecule.

Preferred embodiments are described in the sub-claims.

### Description of the figures

In the drawings schematic views and graphs of spectrum embodiments of the invention are presented.
Figure 1 shows a schematic view of (A) a biosensor principles and related methods of analysis and detection (B) surface-modified ATR element for FTIR detection of biomolecules according to the invention.
Figure 2 shows (A) FTIR-ATR spectrum of a silicon crystal activated and grafted with undecyl trichlorosilane; (B) FTIR-ATR spectrum of a germanium crystal activated and grafted with octadecyl trimethoxysilane.
Figure 3 shows a FTIR-ATR spectrum of a silicon crystal activated and grafted with APTES.
Figure 4 shows a synthetic scheme for the coupling of a spacer-arm (example 2).
Figure 5 shows a FTIR-ATR spectrum of a silicon crystal equipped with the spacer-arm.
Figure 6 shows FTIR spectra of a PE-biotin film exposed to a buffer solution containing streptavidin (125 µg/ml). 100 µg of PE-biotin were applied on the surface of the germanium crystal. A persistaltic pump was used for recirculating the streptavidin aqueous solution into a waterproof vertical ATR flow cell (4 ml/min). Spectra were recorded in the course of the binding of streptavidin on the PE-biotin film. The first ten spectra were recorded every 5 minutes, then every 50 minutes.
Figure 7 shows FTIR-ATR spectra, recorded as a function of time, of a germanium crystal displaying PE-biotin and submitted to a flux of streptavidin solution (125 µg/ml). Intensities of the absorption bands at 1634.7 cm⁻¹ (amide I) and 1543.4 cm⁻¹ (amide II).
Figure 8 shows a graph of calibration obtained by the multivariate analytical technique PLS. Known concentrations of streptavidin vs predicted ones.
Figure 9 shows FTIR-ATR spectra recorded at each step of the construction of the "biotin-streptavidin" sensor. A = crystal grafted with APTES (base line); B = crystal grafted with the spacer-arm (1740 cm⁻¹); C = crystal having fixed the protein (1634.7 and 1543.4 cm⁻¹).
Figure 10 shows a graph of the regeneration of the ATR crystal. A = Crystal with the spacer-arm and the coupled protein, having fixed PE-biotin (1640 cm⁻¹), as indicated by the black arrow; B = crystal with the spacer-arm, recovered after application of a streptavidin solution.
Figure 11 shows a synthetic scheme for the coupling of a spacer-arm (examples 3 and 4).
Figure 12 shows the specificity of ligand/receptor binding. lysozyme (Lys) (130 µg/ml) was added first, then streptavidin (SA) 30 µg/ml was flown in the system. The cell was then washed with 2 mM Hepes, pH 7.5 (hps). Further addition of streptavidin (SA) did not result in any further binding.

### Detailed description of the Invention

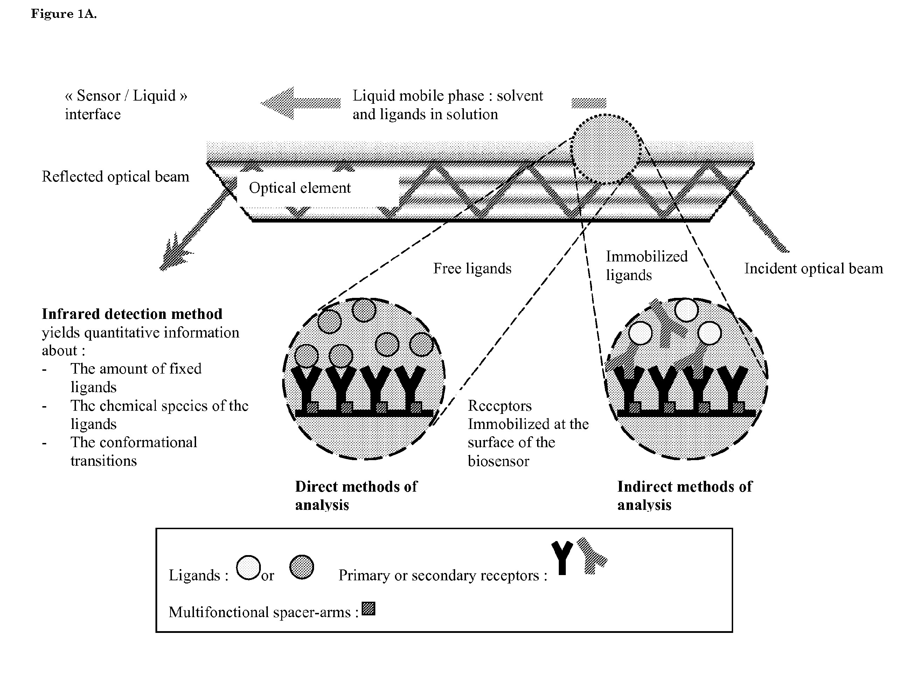

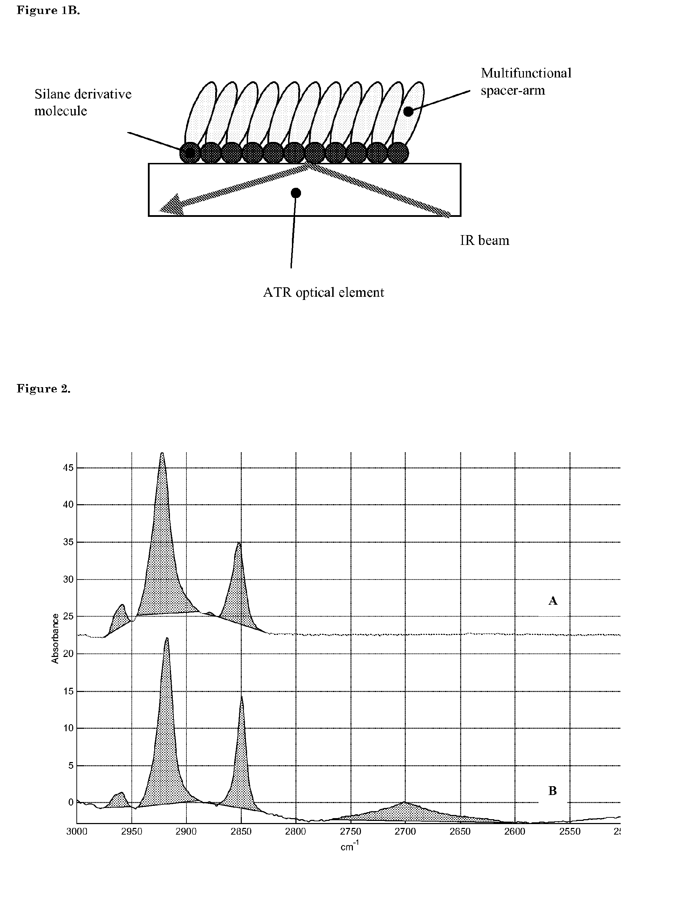

The invention provides a device suitable for the investigation of ligand-receptor interactions, in particular for the investigation of biological molecules and organic components and their interaction with surfaces, consisting of an attenuated total internal reflection element, transparent in the infra-red and of which at least one surface is chemically activated and covalently grafted with a organic molecule able to immobilize a receptor. An embodiment of said device is schematically depicted in figure 1.

In another embodiment the invention provides a method for activating a surface of a attenuated total internal reflection element, by wet chemistry using oxidation / hydroxylation / reduction in an acid or alkaline environment.

In general, the present invention provides a method to activate the surface of an optical element, transparent in the infra-red, particularly a silicon or germanium ATR device, typically a crystal, an optical fiber or a rod made of such materials, and to further covalently fix functionalized organic molecules able to immobilize biological components and molecules, including biological and non-biological receptors, proteins, antibodies, membranes,... This purposely modified ATR element provides a method to study ligand-receptor interactions occurring at the solvent ATR element interface, particularly, at the water-containing media - ATR element interface, by using attenuated total internal reflection (ATR) infra-red (IR) spectroscopy, preferably Fourier transform infra-red spectroscopy (FTIR).

In another embodiment the invention relates to a method, wherein the surface grafting is performed through covalent coupling with a silane derivative. The technique is based on the surface modification, preferably on the oxidation/hydroxylation/reduction, of the ATR element followed by the covalent grafting of organic molecules presenting a reactive moiety at the one terminus for the surface anchorage, typically a silanyl or germanyl functional group, and a functional group at the other terminus for the covalent coupling of biological receptors, either directly, or via multifunctional spacer-arms.

In another embodiment the invention provides a method for studying ligand-receptor interactions, in particular biological molecules or organic components or their interactions or complexations or reactions with biological molecules or organic components or water-soluble molecules at or in the grafted organic molecule, using a surface-activated and covalently-grafted ATR element, comprising the steps of
- installation of the ATR element in a FTIR cell
- conducting a flux of potential ligands, preferably a water-containing solution, on the ATR surface
- analysis of the infra-red spectrum obtained after submitting a FTIR beam through the cell
- regeneration of the ATR surface by application of a solution of free ligand.

In general, the inventive method allows the study of specific ligands interacting with the receptor fixed on the ATR element. The element is placed in a low pressure cell allowing a liquid flux to pass on its surface. A solution of potential ligands to be analyzed is passed through the cell submitted to FTIR beam. Due to the low penetration depth of the evanescent field, ligands in solution do not significantly contribute to the IR spectrum. On the contrary, ligands fixed on the receptors, close to the surface of the ATR element, increase considerably the local concentration at the interface, and contribute to the IR spectrum. The ATR element is preferably a crystal, more preferably having a trapezoidal, fiber or rod shaped geometry. These types of crystals are easy to use and allow good detection.

The quantitative analysis of fixed ligands is an object of the invention.

The time-resolved study of ligand-receptor interaction is a further object of the invention.

The regeneration of the ATR element is another object of the invention.

### Methods for carrying out the invention

### 1. Activation of the A TR element (germanium and silicon)

The activation results from the surface oxidation/hydroxylation by any available technique (physical or chemical), preferably the wet-chemistry technique using a solution of an oxidant in acidic or basic media, such as H₂O₂/H₂SO₄, H₂O₂/TFA, H₂O₂/HF, K₂Cr₂O₇/H₂SO₄, oxone/H₂SO₄, H₂O₂/NH₄OH, or in organic media, such as an organic peracid, Br₂ in solution. The activation may also be carried out by dipping the crystals in sequences of solutions of an oxidant in acidic or basic media. Suitable solutions of an oxidant in acidic or basic media, are e.g. H₂O₂/H₂SO₄, H₂O₂/TFA, H₂O₂/HF, K₂Cr₂O₇/H₂SO₄, oxone/H₂SO₄, H₂O₂/NH₄OH, or e.g. in organic media, such as an organic peracid, Br₂ in a suitable solution, or a combination of these solutions in specific sequences, such as HF in water followed by H₂O₂ in water iterated for several times (e.g. number of repetitions: 3) or NH₄OH/H₂O₂ in water followed by HCl/H₂O₂ in water (e.g. number of repetitions: 1).

The temperature is preferably comprised between -15°C and +150°C and the duration of the treatment comprised between a few seconds to several hours.

### 2. Grafting of a silane derivative on the activated A TR element

The covalent grafting on the activated element is obtained by contacting a solution of silane derivative chosen from: 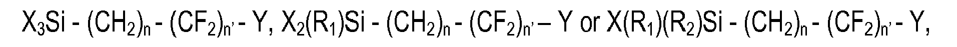 wherein X is halogen, preferably Cl, Br or C₁-C₆ alkoxy, preferably OMe, OEt;
n is 1 to 20;
n' is 0 to 20;
R₁, R₂ are independently C₁-C₆ alkyl;
Y is Me, CF₃, CHF₂, CH₂F, CH=CH₂, CN, CH=O, epoxide, halogen, SH, NH₂, OH, N=C=O, N=C=S, CO₂H or derived esters thereof.

Other suitable parameters in this reaction are preferably:
- solution in organic solvent, such as toluene, dichloromethane, 1,2-dichloroethane, chloroform, acetonitrile
- concentration of 0.01 % to 5%
- temperature of -15°C to 80°C
- reaction time of a few minutes to several hours

### 3. Coupling of a multifunctional spacer-arm

The covalent coupling on the modified ATR element is obtained by contacting a solution of multifunctional molecules of the formula:

Z₁-(CH₂)ₙ-Z₂ wherein n is 2 to 12;

Z₁-CH₂-(O-CH₂-CH₂-)_{n'}-O-CH₂-Z₂ wherein n' is 0 to 5;

wherein Z₁,Z₂ are independently Aryl-N₃, CO₂H and activated forms thereof such as N-hydroxysuccinimidyl ester, CH₂NH₂ and activated derivatives such as N-maleimide, CH₂OH and activated forms such as tosylates, CH₂SH and activated forms such as dithiane derivatives, CH₂N=C=O or CH₂N=C=S.

The same experimental conditions (solvent, concentration, temperature, time) as above apply.
In the case of Z₁, Z₂ equal to Aryl-N₃, light activation is applied ( hλ between 200 and 400 nm).

### 4. Coupling of a silane derivative equipped with the spacer-arm (alternative route to 2. followed by 3.)

The molecules presented in 2. and 3. are reacted to give the following molecules in which X, R₁, R₂, and Z₂ are defined as above :

X₃Si-(CH₂)ₙ-W-(CH2)_{n"}-Z₂

X₃Si-(CH₂)ₙ-W-CH₂-(O-CH₂-CH₂-)_{n"}-O-CH₂-Z₂

wherein n and n" are identical or different from 0-20;
X₃Si can be replaced with X₂(R₁)Si or X(R₁)(R₂)Si;

W is -NHCO-, -CONH-CH₂-, -OCH₂-, -NHCH₂-, -SCH₂-, -S-S-CH₂ or -CH=CH-.

The covalent grafting on the activated ATR element is obtained by contacting a solution of the compounds.

Again the experimental conditions (solvent, concentration, temperature, time) are the same as described under 2 and 3.

### 5. Covalent fixation of a receptor

The ATR element resulting from steps 3. or 4. is placed in contact with an water-containing solution of receptor, preferentially proteins, peptides, membranes,...
The concentration is preferably in the range 10⁻⁶ mgr/ml to 10³ mgr/ml, the temperature is comprised between -15°C and 150°C and the interaction time is from a few seconds to several hours (or up to several hours, if accepted).

The activated functions of the surface modified ATR element react as such; it is the case, for instance, for isocyanate, isothiocyanate, ester of N-hydroxysuccinimide, N-maleimide. The non activated functions, such as free acid, amine, alcohol, are previously activated *in situ* by using the classical methods of peptide synthesis.

### 6. Ligand-receptor interaction and FTIR-ATR detection and quantification

The ATR element obtained in step 5. is placed in the FTIR cell and submitted to a flux of potential ligands, preferably in a water-containing solution.

### 7. Regeneration of the A TR element surface

The fixed ligand (previous step) is displaced from the ATR element surface by the application of a solution of free ligand.

### Examples

### Example 1: surface grafting of molecules on a germanium crystal and their FTIR-ATR detection

### Step 1: surface activation

A germanium crystal was activated by surface treatment with an acid/oxidant mixture at elevated temperature. Typically, a sulfochromic mixture (8 g/l) at 90°C during 1 to 3 hours, preferably 3 hours, was used. The germanium crystal provided with an activated surface is then abundantly rinsed with milliQ-water and dried under a flux of nitrogen.

### Step 2: surface grafting with silane derivatives

The activated germanium crystal of step 1 was exposed to ozone and UV radiation (in an oven) for 30 min, then treated with a solution of alkyl trichlorosilane or alkyl trialkoxysilane in toluene at 20°C. Typically, octadecyl trimethoxysilane (0.05 to 4%, preferably 0.5% in toluene) was reacted during 1 to 16 h, preferably during 2 h to furnish a grafted layer of 4.5 nm in depth (as measured by ellipsometry), corresponding to a water contact angle of 95°. The FTIR-ATR spectrum of this surface-modified crystal showed typical bands between 2850 and 2950 cm⁻¹ due to the CH₂ chain, and a band at 2900 cm⁻¹ due to the terminal CH₃ group (Figure 2B).

Aminopropyl triethoxysilane (APTES) was similarly reacted with the activated germanium crystal (0.5% in toluene, 1-16 h, 20°C) to furnish a grafted layer of 1.2 nm in depth (as measured by ellipsometry), corresponding to a water contact angle of 42°.

Other molecules are undecyltrichlorosilane (C₁₁H₂₃SiCl₃), octadecyltrichlorosilane (C₁₈H₃₇SiCl₃), octadecyltrimethoxysilane (C₁₈H₃₇Si(OCH₃)₃). These molecules are brought in a solution hexadecane and carbon tetrachloride 3/7 (v/v) when trichlorosilanes are used and in a solution of toluene when trialkoxysilanes are used. Grafting was performed in a time period varying from 1 to 16 hours, preferably during 2 hours for alkoxysilanes and 1h30 for the trichlorosilanes. The grafted substrates were subsequently rinsed in a chloroform bath during 3 minutes and then in an acetone bath during 5 minutes.

### Example 2: surface grafting of molecules and functionalized spacers on a silicon crystal and their FTIR-ATR detection (method A)

### Step 1: surface activation

A silicon crystal was surface-activated by treatment with an acid/oxidant mixture at high temperature. Preferably, H₂SO₄/H₂O₂ in ratio 7/3 (v/v) at 150°C during 8 min was used.
Similarly, the crystal was surface-activated by immersion during 5 minutes in a mixture composed out of NH₄OH (25%), oxygenated water H₂O₂ (30%) and MilliQ H₂O in a ratio 1/1/5 (v/v), heated up to 80°C and during agitation, followed by rinsing with MilliQ H₂O and finally an immersion during 5 minutes in a mixture composed out of HCl (15M), H₂O₂ (30%) and MilliQ H₂O in a ratio 1/1/5 (v/v), heated up to 80°C during agitation.

The silicon crystal provided with an activated surface is then abundantly rinsed with MilliQ H₂O and dried under a flux of nitrogen.

### Step 2: surface grafting with silane derivatives

The activated silicon crystal of step 1 was exposed to ozone and UV radiation (in an oven) for 30 min, then treated with a solution of alkyl trichlorosilane or alkyl trialkoxysilane in toluene at 20°C. Typically, undecyl trichlorosilane ( 0.08 % in a mixture composed out of CCl₄ and decane in a ratio 3/7 (v/v)) was reacted during 1 to 16 h at low temperature and low relative humidity, preferably 1.5 h at 12°C and 30%-40% RH, preferably 35%, to furnish a grafted layer of 12 A° in depth (as measured by ellipsometry), corresponding to a water contact angle of 108°. The FTIR-ATR spectrum of this surface-modified crystal showed the characteristic C-H bands around 2900 cm⁻¹ (Figure 2A).

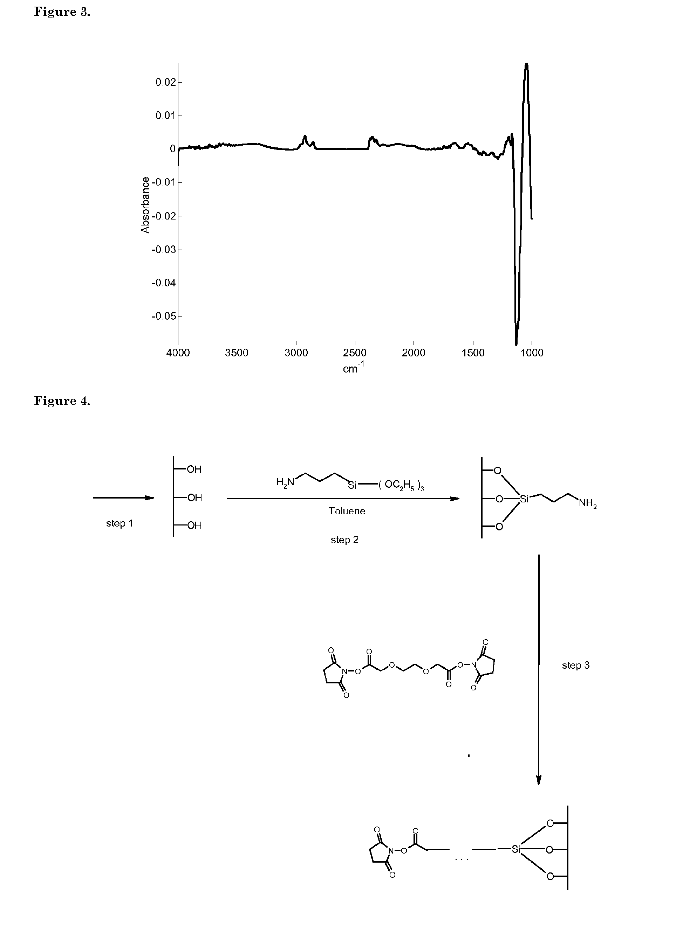

Aminopropyl triethoxysilane (APTES) was similarly grafted on the activated silicon crystal (0.5% in toluene, 20°C; water contact angle: 50°). The FTIR-ATR spectrum of this crystal showed a broad band centered at 3200 cm⁻¹ (NH₂) and sharp bands between 2850 and 2950 cm⁻¹ (CH₂) (Figure 3).

Similarly, other molecules were used: undecenyltrichlorosilane CH₂=CH-C₉H₁₈SiCl₃, octadecyltrichlorosilane C₁₈H₃₇SiCl₃, octadecyltrimethoxysilane C₁₈H₃₇Si(OCH₃)₃. These molecules are brought in a solution and a mixture of hexadecane (for octadecyltrichlorosilane) or decane (for undecenyltrichlorosilane) and of carbon tetrachloride in a ratio 3/7 (v/v) when trichlorosilanes are used and in a solution of toluene when the trialkoxysilanes are used. Grafting is performed during a time period varying from 1 to 16 hours, preferably during 2 hours for the alkoxysilanes and about 1 h30 for the trichlorosilanes.
Subsequently, these grafted silane surfaces are sonicated in a chloroform bath during 3 minutes and subsequently immersed in an acetone bath during 5 minutes.

### Step 3: coupling of a bifunctional spacer-arm

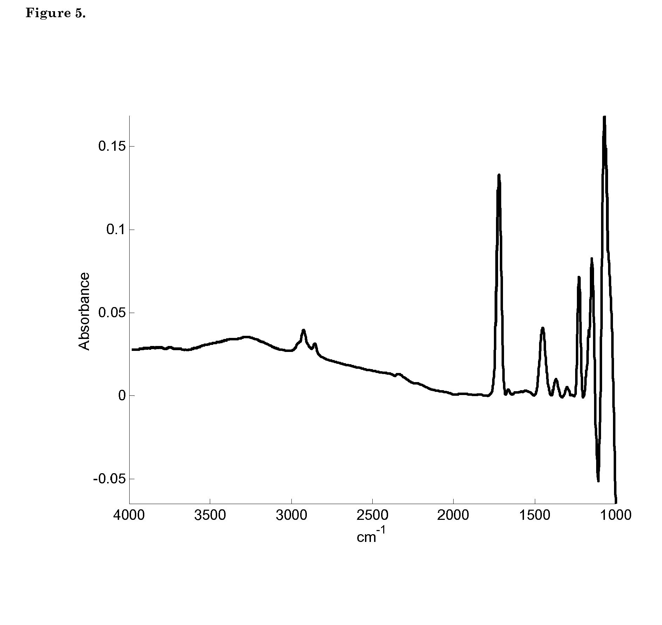

The silicon crystal grafted with APTES, as obtained in step 2, was treated with the bis-activated ester of a α,ω-diacid derivative dissolved in dry organic solvent, at 20°C. Typically, this preferred method used the bis-N-hydoxysuccinimidyl ester of 3,6-dioxaoctane-1,8-dioic acid (0.05 - 2 %, preferably 1% in acetonitrile; 1 to 16 hours) to furnish a crystal surface exposing succinimidyl groups able to covalently fix the receptors of interest. The FTIR-ATR spectrum of this crystal showed a typical carbonyl band at 1700 cm⁻¹ (Figure 5). Rinsing is performed with chloroform, aceton or preferably with acetonitrile or dichloromethane. The activated germanium crystal was consequently rinsed with MilliQ H₂O and dried under a nitrogen flux. An example of a synthetic scheme for the coupling of a spacer arm according to the invention is illustrated in figure 4.

### Example 3: surface grafting of molecules and functionalized spacers on a germanium wafer and their detection by ellipsometry

### Step 1: surface activation

A germanium wafer was activated by surface treatment with a acidic/oxydant sequence at low temperature. Typically, sequences similar to the following one were used : (a) HF(48%) diluted in water (final concentration between 1 % and 20 %, preferably 10%), during 1 to 600 seconds, preferably 10 seconds, at 15° C to 25° C, preferably 20° C and (b) H₂O₂ (30%) diluted in water (final concentration between 1 % and 20 %, preferably 10%), during 1 to 600 seconds, preferably 15 seconds, at 15° C to 25° C, preferably 20° C. The sequence (a), (b) was repeated between 2 and 10 times, preferably 3 times. The germanium wafer provided with an activated surface is then abundantly rinsed with milliQ-water and dried under a flux of nitrogen.

### Step 2: surface grafting with silane derivatives

The activated germanium wafer of step 1 was treated with a solution of alkyl trichlorosilane or alkyl trialkoxysilane in toluene at 20° C. Typically, octadecyl trimethoxysilane (0.5 % in toluene) was reacted during 16 h, then the wafer was rinsed successively in a chloroform bath during 3 min. and in an acetone bath during 5 min., to leave a grafted layer of 4 - 5 nm in depth (as measured by ellipsometry).

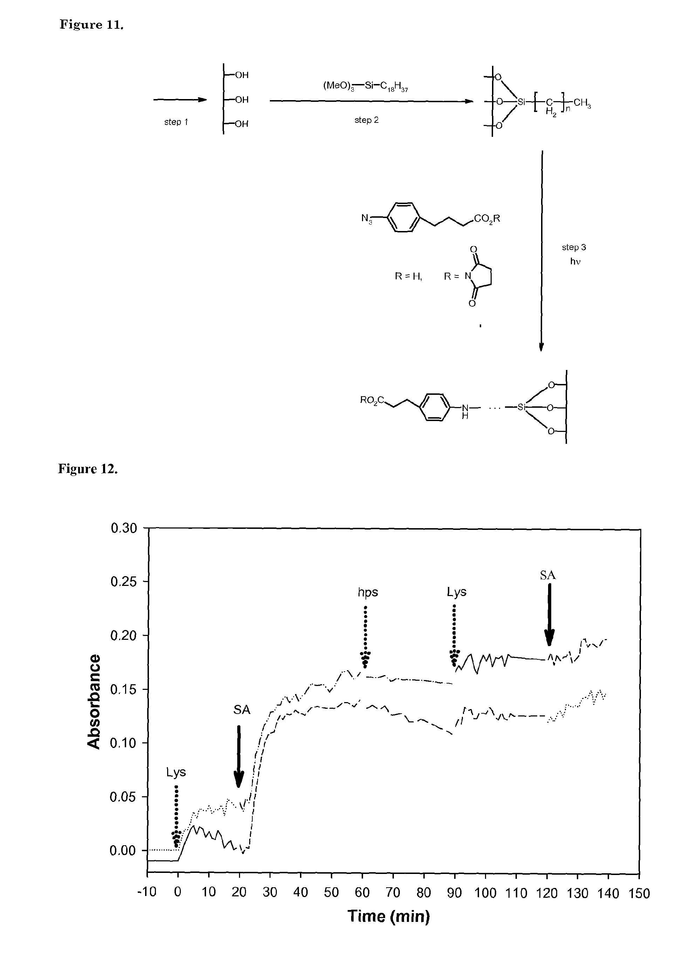

### Step 3: photochemical coupling of a bifunctional spacer-arm (Figure 11)

The germanium wafer grafted with octadecyl groups, as obtained in step 2, was treated with an arylazide under light activation. Typically, this preferred method used 4-(4-azidophenyl)butyric acid (Carnazzi E., Aumalas A., Barberis C., Guillon G., Seyer R., J. Med. Chem. 1994, 37, 1841) or the corresponding N-hydroxysuccinimidyl ester (called activated ester) dissolved in an ether (diethyl ether, or preferably tetrahydrofurane (THF)) (solution at 1 % to 5 %). An aliquot of the previous solution was deposited on the germanium wafer with a pipette and evaporated in the dark in order to obtain an amount of 0.01 mg to 1 mg of coated azide per square centimeter of substrate, preferably 0.1 mg to 0.3 mg. The substrate was then irradiated at 254 nm during 0.1 to 6 hours (preferably 2 h), rinsed successively with chloroform (5 min.) and THF (10 min.), and air dried to furnish a total grafted layer of 7 - 8 nm in depth (as measured by ellipsometry).

### Example 4: surface grafting of molecules and functionalized spacers on a silicon crystal and their FTIR-ATR detection (method B)

### Step 1: surface activation

As described in example 2.

### Step 2: surface grafting with octadecyl trimethoxysilane (OTS)

As described in example 2.

### Step 3: photochemical coupling of a bifunctional spacer-arm (Figure 11)

As described in example 3.

The FTIR - ATR spectrum of the resulted surface-modified crystal showed the characteristic C=O band of the activated ester at 1740 cm⁻¹.

### Example 5: surface coating of a germanium crystal with phosphatidyl ethanolamine coupled to biotin (PE-biotin) and FTIR-A TR quantification of the streptavidin fixation

### Step 1: surface fixation of biotin

A germanium crystal (activated or not) was coated with a membrane made of phosphatidyl ethanolamine coupled to biotin; this PE-biotin layer is stable under an aqueous flux.

### Step 2: surface recognition by streptavidin

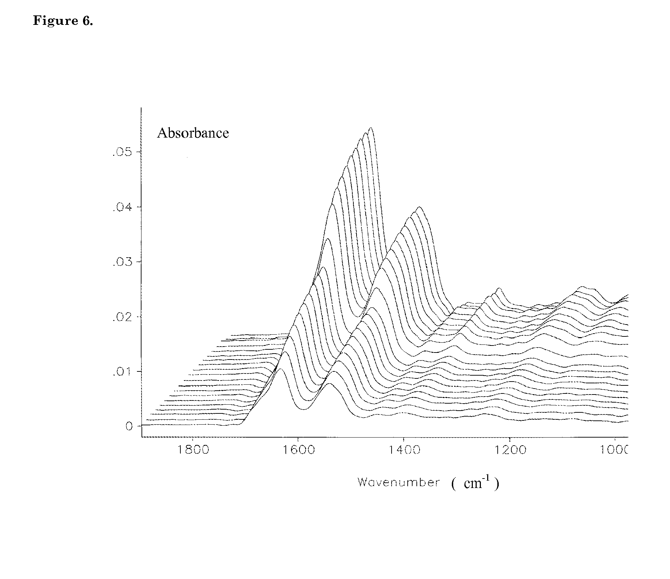

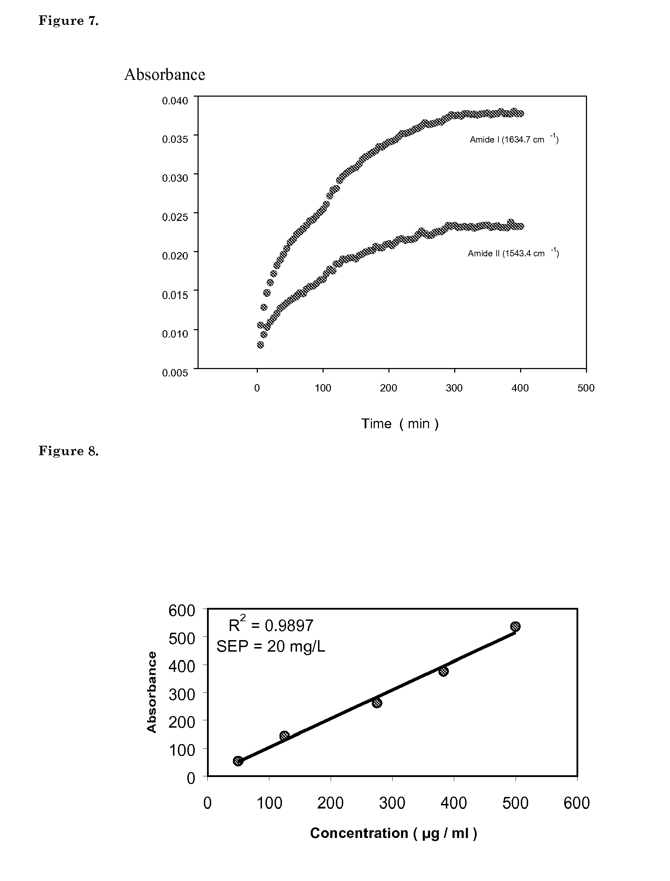

An aqueous flux of a solution of streptavidin (125 µg/ml) was passed on the PE-biotinylated crystal, and FTIR-ATR spectra were recorded every 5 min during 50 min, then every 50 min. The protein contribution was increasingly visible at 1634.7 cm⁻¹ (amide I) and 1543.4 cm⁻¹ (amide II) (Figure 6). After 250 min, the system was saturated when using a streptavidin solution of 250 µg/ml (Figure 7).

### Step 3: quantitative analysis

Although the equilibrium has been reached after 250 min, the streptavidin quantification could be already realized during the ascending phase of the curve (for instance, after 20 min). Five aqueous solutions of streptavidin with concentrations comprised between 50 to 500 µg/ml have been used for calibrating the quantitative analysis of the ligand(biotin)-protein interaction. FTIR-ATR spectra corresponding to the five solutions have been recorded as a function of time of streptavidin adsorption. We have chosen the multivariate analytical technique PLS (partial least squares) to develop a model from the set of reference spectra (Haaland DM, Thomas EV, Anal. Chem., 1988, 60, 1193 and 1202; Dousseau F, Pezolet M, Biochemistry, 1990, 29, 8771). Two spectral domains (1654.7-1602 cm⁻¹ and 1566.4-1499.8 cm⁻¹), representative of the characteristic bands of the protein, have been considered for this calibration. The correlation obtained between the known concentrations of streptavidin and the predicted ones according to the PLS model is illustrated in the Figure 8.

### Example 6: surface modification of a silicon crystal for the fixation of a specific receptor (streptavidin) and regeneration of the surface.

### Step 1: surface activation

as described in example 2

### Step 2: surface grafting with APTES

as described in example 2

### Step 3: coupling of the spacer-arm

as described in example 2

### Step 4: streptavidin fixation

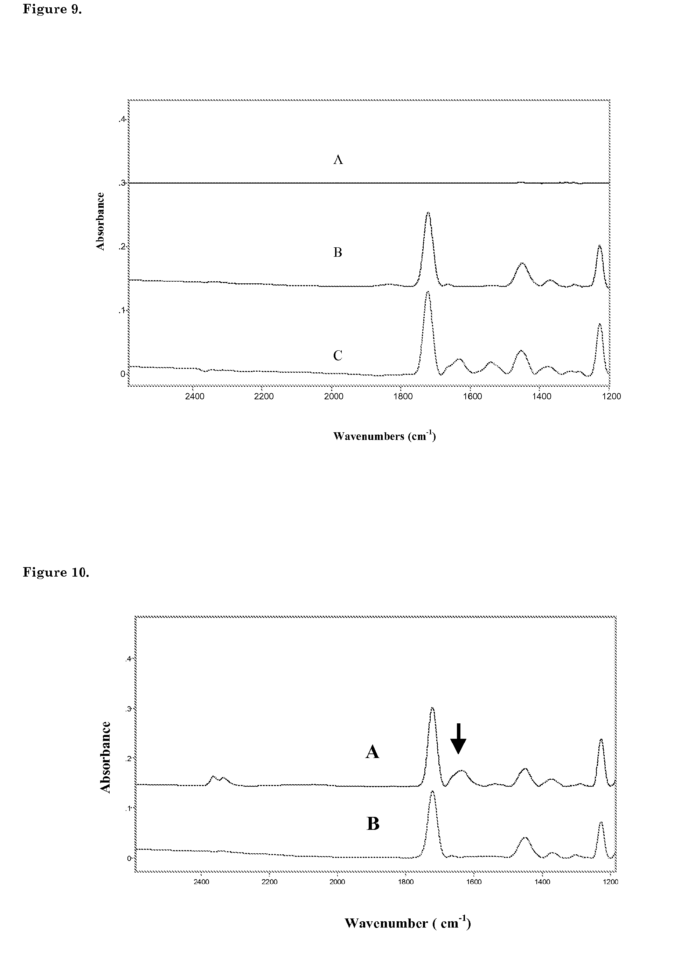

An aqueous solution of streptavidin (250 µg/ml) was placed in contact with the crystal surface resulting from step 3 (20 min, 20°C). The recorded FTIR-ATR spectrum showed the typical bands of amide I (1634.7 cm⁻¹) and amide II (1543.4 cm⁻¹) of the protein (Figure 9). The covalently fixed protein could not be eliminated by washing.

### Step 5: interaction with biotin

A flux of PE-biotin solution was passed on the sensor surface obtained in step 4. The ligand fixation was visible at 1640 cm⁻¹ in the FTIR-ATR spectrum (Figure 10).

### Step 6: surface regeneration

A flux of streptavidin solution was passed on the surface of the sensor obtained in step 5. According to the FTIR-ATR spectrum, the crystal surface of the step 3 has been recovered.

### Example 7: lipid membrane adsorption on an OTS-grafted germanium A TR crystal

### Step 1: surface activation:

as described in example 2

### Step 2: surface grafting with OTS :

as described in example 2

### Step 3: adsorbing a membrane

Lipid vesicles (DDP/PE-biotine 10/1 w:w) 2 mg/ml were incubated overnight in the presence of the coated silicium crystal. After rinsing for 60 min at 0.5 ml/min the crystal was heated at 45°C for 1 hour. The surface was then rinsed again in the same conditions.

### Step 4: measurements

The adsorbed membrane film was shown to bind specifically streptavidin. Concentrations as low as 0.3 µg/ml were readily detected. The recorded FTIR-ATR spectrum showed the typical amide I band of streptavidin (1634 cm⁻¹) characteristic of its beta-sheet secondary structure (Figure 12). Data reported on figure 12 demonstrate that binding is specific. When lysozyme (Lys) was added, no significant binding occurred. When streptavidin (SA) was flown into the system, binding to its receptor present on the surface prepared in step 3 resulted in a large absorbance change. Washing the cell with 2 mM Hepes, pH 7.5 (hps) did not remove the bound streptavindin. Further addition of streptavidin (SA) did not result in any further binding.

The present invention also relates to a device suitable for the investigation of ligand-receptor interactions, in particular for the investigation of an analyte target interaction such as biological and chemical molecules and organic components and their interaction with surfaces, consisting of an attenuated total internal reflection element, transparent in the infra-red and of which at least one surface is chemically activated and covalently grafted with an organic molecule able to immobilize the receptor.

In an embodiment of said device, the organic molecule is a silane derivative of the general formula

X₃Si - (CH₂)ₙ - (CF₂)_{n'} - Y, X₂(R₁)Si - (CH₂)ₙ - (CF₂)_{n'} - Y or X(R₁)(R₂)Si - (CH₂)ₙ -(CF₂)_{n'} - Y,

wherein X is halogen, preferably Cl, Br or C₁-C₆ alkoxy, preferably OMe, OEt;
n is 1 to 20;
n' is 0 to 20;
R₁, R₂ are independently C₁-C₆ alkyl;
Y is Me, CF₃, CHF₂, CH₂F, CH=CH₂, CN, CH=O, epoxide, halogen, SH, NH₂, OH, N=C=O, N=C=S, CO₂H or derived esters thereof.

In an embodiment of said device, the organic molecule is a silane derivative covalently coupled with a multifunctional spacer-arm of the general formula

Z₁-(CH₂)ₙ-Z₂ wherein n is 2 to 12;

Z₁-CH₂-(O-CH₂-CH₂-)_{n'}-O-CH₂-Z₂ wherein n' is 0 to 5;

wherein Z₁,Z₂ are independently chosen from Aryl-N₃ (photoactivable substituent), CO₂H and activated forms thereof such as N-hydroxysuccinimidyl ester), CH₂NH₂ and activated derivatives such as N-maleimide, CH₂OH and activated forms such as tosylates, CH₂SH and activated forms such as dithiane derivatives, CH₂N=C=O or CH₂N=C=S.

In an embodiment of said device, the attenuated total internal reflection element is made from a material chosen from the group consisting of germanium, silicon, ZnSe, ZnS, AM-TIR (an amorphous glass of germanium, selenium and arsenic).

In an embodiment of said device, the attenuated total internal reflection element is a crystal, preferably having a trapezoidal, fiber or rod shaped geometry.

The present invention also relates to the use of a device according to the invention for studying ligand-receptor interactions, in particular biological molecules or organic components or their interactions or complexations or reactions with biological molecules or organic components or water-soluble molecules at or in the grafted organic molecule.

The present invention also relates to a method for the construction of a device according to the invention comprising the steps of:
- surface activation of at least one surface of an attenuated total internal reflection element,
- surface grafting with an organic molecule of the activated surface obtained in the previous step, and
- coupling a receptor via covalent fixation on the organic molecule.

In an embodiment of said method, the surface grafting is performed through covalent coupling with a silane derivative of the general formula

X₃Si - (CH₂)ₙ - (CF₂)_{n'} - Y, X₂(R₁)Si - (CH₂)ₙ - (CF₂)_{n'} - Y or X(R₁)(R₂)Si - (CH₂)ₙ -(CF₂)ₙ, - Y,

wherein X is halogen, preferably Cl, Br or C₁-C₆ alkoxy, preferably OMe, OEt;
n is 1 to 20;
n' is 0 to 20;
R₁, R₂ are independently C₁-C₆ alkyl;
Y is Me, CF₃, CHF₂, CH₂F, CH=CH₂, CN, CH=O, epoxide, halogen, SH, NH₂, OH, N=C=O, N=C=S, CO₂H or derived esters thereof.

In an embodiment, said method further comprises the covalent coupling on the silane derivative of a multifunctional spacer-arm of the general formula

Z₁-(CH₂)ₙ-Z₂ wherein n is 2 to 12

Z₁-CH₂-(O-CH₂-CH₂-)_{n'}-O-CH₂-Z₂ wherein n' is 0 to 5

wherein Z₁,Z₂ are independently Aryl-N₃, CO₂H and activated forms thereof such as N-hydroxysuccinimidyl ester), CH₂NH₂ and activated derivatives such as N-maleimide, CH₂OH and activated forms such as tosylates, CH₂SH and activated forms such as dithiane derivatives, CH₂N=C=O or CH₂N=C=S.

In an embodiment of said method, the organic molecule is a silane derivative covalently linked with a spacer-arm, the general formula of said organic molecule being:

X₃Si-(CH₂)ₙ-W-(CH₂)_{n"}-Z₂

X₃Si-(CH₂)ₙ-W-CH₂-(O-CH₂-CH₂-)_{n"}-O-CH₂-Z₂

wherein n and n" are identical or different from 0-20;
X₃Si can be replaced with X₂(R₁)Si or X(R₁)(R₂)Si;

W is -NHCO-, -CONH-CH₂-, -OCH₂-, -NHCH₂-, -SCH₂-, -S-S-CH₂ or -CH=CH-.

In an embodiment of said method, a receptor is immobilized on the organic molecule.

The present invention also relates to a device obtainable by a method according to the invention.

The present invention also relates to a method for activating a surface of an attenuated total internal reflection element, by wet chemistry using oxidation/hydroxylation/reduction in an acid or alkaline environment.

In an embodiment, said method comprises the activation of a silicon crystal by the treatment of a surface thereof with a mixture H₂SO₄/H₂O₂ in water.

In an embodiment, said method comprises the activation of a germanium crystal by the treatment of a germanium surface with a sulfochromic mixture.

In an embodiment, said method comprises the activation of a germanium crystal by treatment of the germanium surface with a mixture NH₄OH/H₂O₂ in water followed by HCl/H₂O₂ in water.

In an embodiment, the surface treatment is performed by dipping the crystals in sequences of solutions of an oxidant in acidic or basic media and optionally iterated.

In an embodiment, the surface treatment is performed by the sequence NH₄OH/H₂O₂ in water followed by HCl/H₂O₂ in water and optionally iterated.

In an embodiment, the surface treatment is performed by the sequence HF in water followed by H₂O₂ in water and optionally iterated for several times, such as 3 times.

The present invention also relates to a method for studying ligand-receptor interactions, in particular biological molecules or organic components or their interactions or complexations or reactions with biological molecules or organic components or water-soluble molecules at or in the grafted organic molecule, using a device according to the invention, comprising the steps of
- installation of said device in a FTIR cell
- conducting a flux of potential ligands, preferably a water-containing solution, on said device surface
- analysis of the infra-red spectrum obtained after submitting a FTIR beam through said cell
- regeneration of said device surface by application of a solution of free ligand.

## Claims

1. Method for activating a surface of a germanium attenuated total internal reflection element, for direct grafting of an organic component on said surface, by wet chemistry using oxidation/hydroxylation/reduction in an acid or alkaline environment.

2. Method according to claim 1 for the activation of a germanium crystal by the treatment of a germanium surface with a sulfochromic mixture.

3. Method according to claim 1 for the activation of a germanium crystal by treatment of the germanium surface with a mixture NH₄OH/H₂O₂ in water followed by HCl/H₂O₂ in water.

4. Method according to claim 1 whereby the surface treatment is performed by dipping the crystals in sequences of solutions of an oxidant in acidic or basic media and optionally iterated.

5. Method according to claim 1 or 4 whereby the surface treatment is performed by the sequence NH₄OH/H₂O₂ in water followed by HCl/H₂O₂ in water and optionally iterated.

6. Method according to claim 1 or 4 whereby the surface treatment is performed by the sequence HF in water followed by H₂O₂ in water and optionally iterated for several times, such as 3 times.
